**Europäisches Patentamt**

**European Patent Office**

**Office européen des brevets**

(11) Veröffentlichungsnummer: **0 314 856 B1**

# EUROPÄISCHE PATENTSCHRIFT

(45) Veröffentlichungstag der Patentschrift :
**05.06.91 Patentblatt 91/23**

(51) Int. Cl.$^5$ : **A61M 5/14**

(21) Anmeldenummer : **88101274.4**

(22) Anmeldetag : **29.01.88**

(54) **Y-Rohrformstück für Infusionsgeräte.**

(30) Priorität : **04.11.87 DE 8714659 U**

(43) Veröffentlichungstag der Anmeldung :
**10.05.89 Patentblatt 89/19**

(45) Bekanntmachung des Hinweises auf die
Patenterteilung :
**05.06.91 Patentblatt 91/23**

(84) Benannte Vertragsstaaten :
**AT CH DE ES FR IT LI NL SE**

(56) Entgegenhaltungen :
**US-A- 4 048 995**
**US-A- 4 219 022**
**US-A- 4 596 557**

(73) Patentinhaber : **CLINICO INFUSIONSTECHNIK**
**GmbH**
**Robert-Koch-Strasse 5**
**W-6430 Bad Hersfeld (DE)**

(72) Erfinder : **Heinzerling, Walter Chr.**
**Wacholderweg 9**
**W-6430 Bad Hersfeld (DE)**
Erfinder : **Schadt, Wolfgang**
**Hölderlinstrasse 25**
**W-6442 Rotenburg 1 (DE)**

(74) Vertreter : **von Raffay, Vincenz, Dipl.-Ing. et al**
**Patentanwälte Raffay & Fleck Postfach 32 32**
**17**
**W-2000 Hamburg 13 (DE)**

EP 0 314 856 B1

## Beschreibung

Die Erfindung betrifft ein Y-Rohrformstück nach dem Oberbegriff des Patentanspruches 1. Derartige Y-Rohrformstücke sind bekannt. Die Zuspritzung von Medikamenten während der Infusion erfolgt über den Injektionspfropfen aus Latex oder dergleichen. Bei der Erstfüllung eines Infusionsbesteckes mit Y-Rohrformstück besteht die Gefahr, daß ein Restluftpolster im Bereich des Injektionspfropfens verbleibt. Dieses ist gefährlich, da Lufteinschlüsse in der Infusionslösung auf jeden Fall vermieden werden müssen.

Der Erfindung liegt daher die Aufgabe zugrunde, ein Y-Rohrformstück nach dem Oberbegriff des Patentanspruches 1 bzw. 2 zu schaffen, das die Bildung derartiger Restluftpolster im Bereich des Injektionspfropfens verhindert.

Diese Aufgabe wird bei unterschiedlichen Ausführungsformen durch das Kennzeichen des Anspruches 1 bzw. des Anspruches 2 gelöst.

Erfindungsgemäß wird ein Umlenkungs- (Patentanspruch 1) bzw. ein Umgehungskanal (Patentanspruch 2) geschaffen, der sicherstellt, daß sich im Bereich des Injektionspfropfens, d.h. in dem Zuspritzkanal, kein Restluftpolster bilden kann. In diesem Bereich wird bei der Erstbefüllung für eine entsprechende Strömung gesorgt, so daß der Verbleib von Luft hier ausgeschlossen ist.

Bei der einen Ausführungsform wird die gesamte, in den Einlaufkanal des Y-Rohrformstückes eingegebene Injektionsflüssigkeit in den Bereich des Injektionspfropfens umgeleitet, so daß die gesamte Strömung auch durch diesen Bereich erfolgt. Es kann hier also kein Totraum, in dem sich Luft ansammeln kann, verbleiben.

Bei der anderen Ausführungsform wird sichergestellt, daß sich in dem Zuspritzkanal stromaufwärts von der Einmündung des Einlaufkanals ein Unterdruck bildet, der für eine Teilströmung der Injektionsflüssigkeit durch diesen Bereich bis in den Bereich des Injektionspfropfens und aus diesem zurück durch den Umgehungskanal in den entsprechenden Teil des Zuspritzkanals in Richtung auf den Auslauf sorgt.

Im folgenden wird die Erfindung unter Hinweis auf die Zeichnung anhand der beiden Ausführungsbeispiele näher erläutert.

Es zeigt:

Fig. 1 einen schematischen Querschnitt durch die eine Ausführungsform des Y-Rohrformstückes; und

Fig. 2 einen entsprechenden Schnitt durch die andere Ausführungsform des Y-Rohrformstückes.

Die Rohrformstücke der beiden Ausführungsformen weisen einen Zuspritzkanal 5 auf, in den seitlich ein Einlaufkanal 3 führt. Der Zuspritzkanal ist durch einen Injektionspfropfen 2 aus Latex oder einem ähnlichen Material verschlossen und zwar mit Hilfe eines Verschlusses 7. Der Auslaufkanal ist mit 1 bezeichnet.

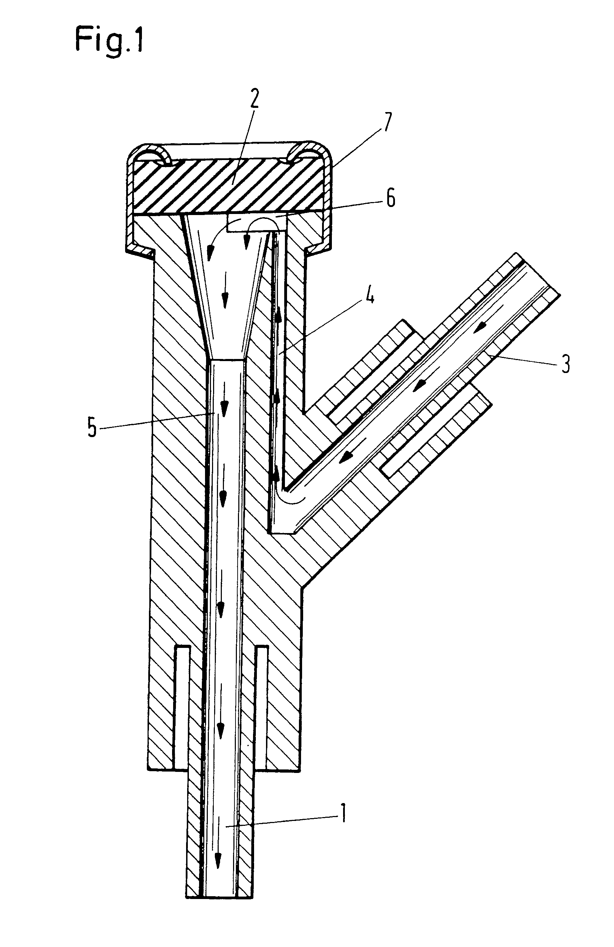

Bei der Ausführungsform nach Fig. 1 ist der Einlaufkanal 3 nicht direkt mit dem Zuspritzkanal 5 verbunden, sondern es ist ein Umlenkkanal 4 vorgesehen, der in den Bereich des Injektionspfropfens 2 führt und so die Verbindung zu dem Zuspritzkanal herstellt.

Es ist erkennbar, daß die Strömung der Injektionsflüssigkeit bei der Erstbefüllung in den Bereich angrenzend an den Injektionspfropfen 2 führt und so verhindert, daß sich in diesem Bereich ein Restluftpolster bilden kann.

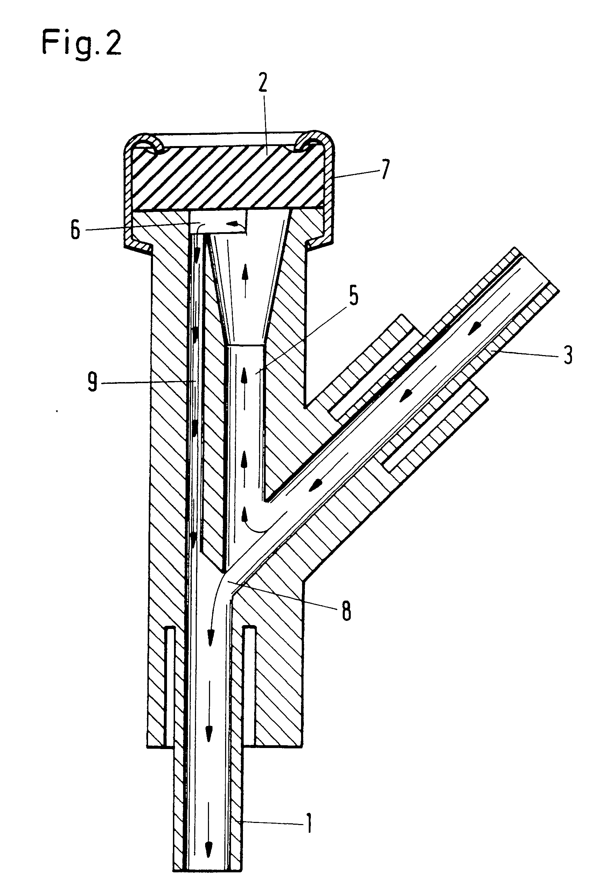

Bei der Ausführungsform nach Fig. 2 führt der Einlaufkanal 3 direkt in den Zuspritzkanal 5. Im Einmündungsbereich ist eine Drosselstelle 8 vorgesehen. Parallel zu dem Zuspritzkanal 5 ist ein Umgehungskanal 9 vorgesehen, der eine Verbindung zwischen dem Bereich angrenzend an den Injektionspfropfen 2 und einem Punkt stromab von der Drosselstelle 8 herstellt.

Bei der Erstbefüllung entsteht durch die Drosselstelle 8 stromab von dieser ein Unterdruck, so daß ein Teil der Strömung durch den Umgehungskanal 9 erfolgt, wie es in Fig. 2 durch Pfeile angedeutet ist. Auch bei dieser Ausführungsform stellt die Strömung durch den Injektionsbereich sicher, daß sich hier kein Restluftpolster bilden kann.

Bei beiden Ausführungsformen ist die Verbindung zwischen dem Injektionsbereich und dem Umlenkkanal 4 bzw. dem Umgehungskanal 9 mit 6 bezeichnet.

## Ansprüche

1. Y-Rohrformstück für Infusionsgeräte mit einem Einlaufkanal (3) für die Infusionslösung und einem Zuspritzkanal (5) mit Injektionspfropfen (2) zur Zuspritzung von Medikamenten und einem gemeinsamen Auslaufkanal (1), dadurch gekennzeichnet, daß der Einlaufkanal (3) durch einen parallel zum Zuspritzkanal (5) verlaufenden Umlenkkanal (4) mit dem an den Injektionspfropfen angrenzenden Bereich des Zuspritzkanals (5) verbunden ist.

2. Y-Rohrformstück für Infusionsgeräte mit einem Einlaufkanal (3) für die Infusionslösung und einem Zuspritzkanal (5) mit Injektionspfropfen (2) zur Zuspritzung von Medikamenten und einem gemeinsamen Auslaufkanal (1), dadurch gekennzeichnet, daß im Bereich der Einmündung des Einlaufkanals (3) in den Zuspritzkanal (5) eine Drosselstelle (8) vorgesehen ist, und daß der an den Injektionspfropfen (2) angrenzende Bereich des Zuspritzkanals (5) durch einen Umgehungskanal (9) mit dem Zuspritzkanal stromab von der Drosselstelle (8) verbunden ist.

## Claims

1. Y-pipe fitting for infusion equipment with an intake duct (3) for the infusion solution and an injection duct (5) with injection plug (2) for injecting medicaments and a common discharge duct (1), characterized in that the intake duct (3) is connected by a deflecting duct (4) running parallel to injection duct (5) with the area of the latter adjacent to the injection plug.

2. Y-pipe fitting for infusion equipment with an intake duct (3) for the infusion solution and an injection duct (5) with injection plug or injecting medicaments and a common discharge duct (1) characterized in that in the vicinity of the issuing point of the intake duct (3) into the injection duct (5) is provided a constriction (8) and that the area of the injection duct (5) adjacent to the injection plug (2) is connected by a bypassing duct (9) to the injection duct downstream of constriction (8).

## Revendications

1. Pièce tubulaire en forme de Y pour appareils de perfusion comportant un passage d'entrée (3) destiné à la solution de perfusion et un canal d'injection (5) muni d'un opercule d'injection (2) destiné à l'injection de médicaments, et un canal de sortie (1) commun, caractérisé par le fait que le passage d'entrée (3) est relié par un passage de déviation (4) s'étendant parallèlement au canal d'injection (5) à la zone du canal d'injection (5) qui est située à proximité immédiate de l'opercule d'injection.

2. Pièce tubulaire en forme de Y pour appareils de perfusion comportant un passage d'entrée (3) destiné à la solution de perfusion et un canal d'injection (5) muni d'un opercule d'injection (2) destiné à l'injection de médicaments, ainsi qu'un canal de sortie commun (1), caractérisé par le fait que dans la zone où le canal d'entrée (3) débouche dans le canal d'injection (5) on prévoit un étranglement (8) et que la zone du canal d'injection située à proximité immédiate de l'opercule d'injection (2) est reliée au canal d'injection (5) par un passage de contournement (9), en aval de l'étranglement (8).

# Fig.1

# Fig.2